Europäisches Patentamt

European Patent Office

Office européen des brevets

(19)

(11) Publication number : **0 427 572 B1**

(12) **EUROPEAN PATENT SPECIFICATION**

(45) Date of publication of patent specification :
27.04.94 Bulletin 94/17

(51) Int. Cl.⁵ : **C07C 215/76, C07C 213/08**

(21) Application number : **90312304.0**

(22) Date of filing : **09.11.90**

(54) **Process for preparing N-alkylaminophenols and N,N-dialkylaminophenols.**

(30) Priority : **10.11.89 JP 293392/89**
**10.11.89 JP 293393/89**

(43) Date of publication of application :
**15.05.91 Bulletin 91/20**

(45) Publication of the grant of the patent :
**27.04.94 Bulletin 94/17**

(84) Designated Contracting States :
**DE FR GB IT NL**

(56) References cited :
**EP-A- 0 181 505**
**DE-A- 3 039 085**
**DERWENT PUBLICATIONS LTD., London**
**Questel Telesystems (WPI), DW 7418**
**PATENT ABSTRACTS OF JAPAN, unexamined**
**applications, C field, vol. 10, no. 214, July 25,**
**1986 THE PATENT OFFICE JAPANESE GOV-**
**ERNMENT page 79 C 362**

(73) Proprietor : **SUMITOMO CHEMICAL**
**COMPANY, LIMITED**
**5-33 Kitahama 4-chome Chuo-ku**
**Osaka-shi Osaka (JP)**

(72) Inventor : **Maki, Hiroshi**
**1353-4, Shiizu**
**Ichihara-shi, Chiba (JP)**
Inventor : **Kawasaki, Michihiro**
**1-13-15, Sakuradai**
**Ichihara-shi, Chiba (JP)**
Inventor : **Shimizu, Hiroshi**
**1-9, Yushudainishi**
**Ichihara-shi, Chiba (JP)**
Inventor : **Ito, Yoshiaki**
**1-9, Yushudainishi**
**Ichihara-shi, Chiba (JP)**

(74) Representative : **Diamond, Bryan Clive et al**
**Gee & Co. Chancery House Chancery Lane**
**London WC2A 1QU (GB)**

EP 0 427 572 B1

## Description

This invention relates to a process for preparing an N-alkylaminophenol or a N,N-dialkylaminophenol by subjecting an aminophenol to reductive alkylation with an aldehyde or a ketone in the presence of an organic solvent, a catalyst for reduction, and hydrogen.

N-Alkylaminophenols are of extreme importance in industry as intermediates for heat-sensitive or pressure-sensitive dyes, xanthene dyes, fluorescent dyes, etc.

It is known to prepare an N-alkylaminophenol or a N,N-dialkylaminophenol by reductive alkylation of an alkylaminophenol and an aldehyde or a ketone in the presence of an organic solvent, a catalyst for reduction, and hydrogen.

However, the conventional processes have the following disadvantage. As the conventional noble metal catalysts for use in the reductive alkylation, such as platinum or palladium catalysts, have an ability to reduce an aromatic ring when used as such, nuclear hydrogenation of the aromatic ring may be caused as a side reaction depending on the reaction conditions, resulting in a decrease in the yield of the desired N-alkylaminophenols. Industrially, since these catalysts are expensive, generally it is necessary to use them repeatedly, but this has the following industrially serious problems in addition to the nuclear hydrogenation of aromatic ring. First the aminophenol and the aldehyde or ketone are condensed to form a heavy matter. Also the aldehyde or ketone is reduced to increase the by-production of an alcohol.

Of the problems, concerning the by-production of an alcohol, for example, JP-A-57-165349, JP-A-58-26844, and JP-A-58-194843 disclose techniques for suppressing the by-production of an alcohol by adding a solid sulfur compound during the course of the reductive alkylation, or using a platinum sulfide catalyst. (The term "JP-A" as used herein means an "unexamined published Japanese patent application".)

However, though these techniques can suppress the by-production of an alcohol to a some extent, the reductive alkylation as a main reaction is also suppressed at the same time; thus there is a problem that an unstable Schiff's base of an aminophenol is condensed to form a heavy matter. These techniques also have disadvantages that when the catalyst is used repeatedly, the reaction can be controlled only with difficulty due to elimination of sulfur from the catalyst.

Further almost no conventional techniques have been mentioned about a technique for suppressing nuclear hydrogenation of an aromatic ring, especially no technique for suppressing nuclear hydrogenation of an aromatic ring in the preparation of an N-alkylaminophenol or of a N,N-dialkylaminophenol has been known.

An object of this invention is to provide an industrially advantageous process for preparing an N-alkylaminophenol or a N,N-dialkylaminophenol which can suppress undesirable side reactions such as nuclear hydrogenation of an aromatic ring and reaction which forms heavy matters as described above, and which enables a catalyst to be used repeatedly with a high yield of the desired products.

The present inventors have found that the above-described object can be attained by use of a platinum or palladium catalyst containing a specific metal element selected in the Periodic Table, or a platinum or palladium catalyst which has been contact treated with a solution containing a specific metal element.

Thus this invention concerns a process for preparing an N-alkylaminophenol or a N,N-dialkylaminophenol, which comprises subjecting an aminophenol to reductive alkylation with an aldehyde or a ketone in the presence of an organic solvent, a catalyst for reduction, and hydrogen, wherein the catalyst for reduction comprises platinum and at least one metal element selected from metal elements belonging to the IB group, IIB group, IVB group, VB group and VIB group of the Periodic Table, supported on activated carbon, or comprises palladium and at least one metal element selected from metal elements belonging to the IB group, IIB group, IVB group, VB group and VIB group of the Periodic Table, supported on activated carbon.

Aminophenols which can be used in this invention include, for example, o-aminophenol, m-aminophenol and p-aminophenol.

Aldehydes which can be used in this invention include, for example, aliphatic aldehydes such as formaldehyde, acetaldehyde, propionaldehyde, butyraldehyde, and isovaleraldehyde; cyclic aldehydes such as cyclohexanecarboxyaldehyde and furfural; and aromatic aldehydes such as benzaldehyde and p-tolualdehyde.

Ketones which can be used in this invention include, for example, aliphatic ketones such as acetone, 2-butanone, and 4-methyl-2-pentanone; cyclic ketones such as cyclopentanone and cyclohexanone; and aromatic ketones such as acetophenone and p-methylacetophenone.

N-Alkylaminophenols which can be obtained by means of this invention include, for example, N-monoalkylaminophenols such as N-ethylaminophenol, N-propylaminophenol, N-butylamino-phenol, N-cyclohexylaminophenol, N-benzylaminophenol, and N-isopropylaminophenol; and N,N-dialkylaminophenols such as N,N-diethylaminophenol, N,N-dibutylaminophenol, N-ethyl-N-isobutylaminophenol, and N-ethyl-N-isoamylaminophenol.

Organic solvents which can be used in this invention include, for example, aliphatic alcohols such as methanol and ethanol.

A catalyst for reduction which can be used in this invention is a platinum or palladium catalyst containing a specific metal element selected in the Periodic Table, or a platinum or palladium catalyst which has been contact treated with a solution containing a specific metal element selected in the Periodic Table.

The term "metal element selected in the Periodic Table" is intended to be at least one metal element selected from the IB group, IIB group, IVB group, VB group, and VIB group of the Periodic Table.

Typical examples of such metallic elements include Cu, Zn, Cd, Sn, Pb, As, Sb, Se and Te, with Pb, Te, Cu, and As being preferable.

The content of the metal in the catalyst for reduction is preferably in the range of from 0.001 to 0.5 part by weight, more preferably from 0.005 to 0.2 part by weight, per part by weight of platinum or palladium. If it is less than 0.001 part by weight, the effect of suppressing nuclear hydrogenation of an aromatic ring may be insufficient. On the other hand, if it exceeds 0.5 part by weight, the activity of the catalyst for reduction on the reductive alkylation which is the main reaction may be decreased so that the aminophenol and the aldehyde or ketone are likely to be condensed to form a heavy matter.

In the present invention, methods using the platinum or palladium catalyst which has been contact treated with a solution containing a specific metal element selected in the Periodic Table as described above are as follows:

(1) a method wherein a catalyst slurry obtained by adding a metal salt containing a metal element selected in the Periodic Table to a slurry comprising an organic solvent and a platinum or palladium catalyst supported on activated carbon for use in the reductive alkylation is used for the reductive alkylation as is, or
(2) a method wherein a solid catalyst obtained by filtering the catalyst slurry mentioned in (1) is used for the reductive alkylation.

Typical embodiments will be mentioned as follows:

The method (1) can be carried out by charging a fresh catalyst, or the catalyst which has been used in the reaction and recovered, together with the organic solvent, and then adding the metal salt alone or as a solution in an organic solvent with stirring. This procedure may be carried out either under an inert gas atmosphere, $N_2$-pressurized atmosphere, or $H_2$-pressurized atmosphere. The temperature and the time of the treatment are not specifically limited, but generally the treatment is sufficiently carried out at from room temperature to 90°C for from 5 minutes to 2 hours. After this contact treatment, it is preferable to treat the resulting catalyst at room temperature and at atmospheric pressure taking into consideration the reductive alkylation to be carried out thereafter.

The method (2) can be carried out by charging a fresh catalyst, or the catalyst which has been used in the reaction and recovered, together with the organic solvent, adding the metal salt alone or as a solution with stirring, and after stirring, recovering a solid catalyst by filtration. This operation may be carried out either under an inert gas atmosphere at atmospheric pressure or under $N_2$-pressurised atmosphere. The temperature and the time of the operation are not specifically limited, but generally the treatment is sufficiently carried out at from room temperature to 90°C for from 5 minutes to 2 hours.

In the catalyst for reduction which has been contact treated by the method (1) or (2), almost the whole amount of the metal element used in the contact treatment is contained therein.

In the case of the method (2), the solvent to be used for the formation of the catalyst slurry is not necessarily the organic solvent to be used for the reductive alkylation, but it is preferably the same solvent as that used for the reductive alkylation or water,taking into consideration the contamination of the reaction system of the reductive alkylation.

As the metal salt used for obtaining the catalyst slurry, oxides, chlorides, bromides, sulfates, nitrates, phosphates, acetates and oxalates of the metal elements selected in the Periodic Table can be used, but from the standpoint of uniform adsorption of the metal element on the platinum or palladium catalyst, acetates are most preferable.

The amount of the metal element to be used for the contact treatment is preferably in the range of from 0.001 to 0.5 part by weight, more preferably from 0.005 to 0.2 parts by weight, per part by weight of platinum or palladium. If it is less than 0.001 part by weight, the effect of suppressing nuclear hydrogenation of an aromatic ring may be insufficient. On the other hand, if it exceeds 0.5 part by weight, the activity of the catalyst for reduction on the reductive alkylation which is the main reaction may be decreased so that the aminophenol and the aldehyde or ketone are likely to be condensed to form a heavy matter.

The reaction of this invention is a reaction which gives an N-alkylaminophenol or a N,N-dialkylaminophenol from an aminophenol and an aldehyde or a ketone by reductive alkylation in the presence of the above-described organic solvent, hydrogen and catalyst for reduction. The reaction may be carried out by feeding the aminophenol, organic solvent and the catalyst for reduction under hydrogen pressure, continuously feeding the aldehyde or ketone, or feeding them all at once to bring about the reaction. However, from the viewpoint of carrying out the reaction smoothly, the aldehyde or ketone is preferably fed continuously, and it is more preferable to continuously add a small amount of an organic carboxylic acid such as acetic acid corresponding to the feeding of the aldehyde or ketone. It is sufficient that the reaction temperature is in the range of from room temperature to 150°C, and the reaction pressure may be in the range of from 2 to $29.4 \times 10^5$ Pa (2 to 30 kg/cm$^2$G).

The amount of the catalyst for reduction to be used is preferably in the range of from 0.0001 to 0.02 part by weight, more preferably from 0.001 to 0.01 part by weight, as platinum or palladium in the catalyst, per part by weight of the starting material aminophenol. If it is less than 0.0001 part by weight, the rate of the main reaction becomes unduly slow, and the formation of heavy matters as a side reaction may be accelerated. Conversely, if it is more than 0.02 part by weight, the reduction of the aromatic ring may be accelerated. The catalyst for reduction may be used only once, but usually it is used repeatedly on an industrial scale. Particularly, since almost the whole amount of the metal element selected in the Periodic Table remains in the catalyst used herein even after the catalyst has been used once, no special treatment is needed in the case that this catalyst for reduction is used repeatedly, and the effect of this invention can be maintained. In the case of repeated use since there is a loss of the catalyst by finely dividing and a loss of the recovery by filtration, a small amount of the fresh catalyst is supplemented as occasion may demand and used for the next reductive alkylation.

It is also possible to add the metal element selected in the Periodic Table in such an amount as to meet the fresh catalyst to be supplemented.

As described above, an N-alkylaminophenol or a N,N-dialkylaminophenol can be obtained by the process for preparing an N-alkylaminophenol by subjecting an aminophenol to reductive alkylation with an aldehyde or a ketone in the presence of an organic solvent, a catalyst for reduction, and hydrogen according to the present invention while suppressing side reactions such as nuclear hydrogenation of an aromatic ring and a reaction to form heavy matters as described above with a high yield of the desired products. Especially, when the catalyst for reduction is used repeatedly, the product can be prepared with an industrially outstanding advantage.

Now, the present invention will be described in more detail with reference to working examples.

## Example 1

In a 500 cc-volume SUS-made autoclave equipped provided with a stirrer were charged 32.7 g (0.30 mole) of m-aminophenol, 185.5 g of methanol, and 1.6 g of a commercially available catalyst comprising 5% by weight of platinum supported on activated carbon containing 0.1 part by weight of lead per part by weight of platinum, and 67.6 g of a methanol solution containing 45% by weight of acetaldehyde (acetaldehyde content: 0.69 mole) and 0.20 g (0.0033 mole) of acetic acid were introduced over a period of 1 hour under constant conditions of 40°C and $9.8 \times 10^5$ Pa (10 kg/cm$^2$G) of hydrogen pressure. After completion of the introduction of acetaldehyde, the mixture was maintained at the same temperature for an additional 150 minutes and allowed to cool. The catalyst was removed by filtration, and the resulting filtrate was analyzed by gas chromatography, liquid chromatography, and GPC (gel permeation chromatography). As a result, it was found to be 100% of the conversion of m-aminophenol (calculated by m-aminophenol consumed/m-aminophenol charged x 100), 95.9% of the selectivity of 3-(N,N-diethylamino)phenol, 0.5% of the selectivity of a nuclear hydrogenated product of benzene ring (nuclear hydrogenated product: 3-(N,N-diethylamino)-2-hexen-1-one), and 2.1% of the selectivity of heavy matter (values of all selectivity being relative to m-aminophenol).

## Examples 2 to 4

Using a catalyst obtained after being used in Example 1 and then recovered, the reaction was carried out in a similar manner to Example 1. Afterwards, the reaction was carried out using the catalyst recovered again and again, the results being shown in Table 1.

## Table 1

| Example | Number of times of catalyst used | Yield of DEMP (%) | Yield of nuclear hydrogenated product (%) | Yield of heavy matter (%) |
|---------|----------------------------------|-------------------|-------------------------------------------|----------------------------|
| 2 | 2 | 95.2 | 2.5 | 1.7 |
| 3 | 3 | 95.8 | 2.1 | 2.0 |
| 4 | 4 | 95.1 | 2.8 | 2.1 |

Note: DEMP: 3-(N,N-Diethylamino) phenol

All the conversion of m-aminophenol was 100%.

### Examples 5 to 8

The reaction of Examples 1 to 4 was repeated, except that 1.6 g of a commercially available catalyst comprising 5% by weight of platinum supported on activated carbon containing 0.06 part by weight of tellurium per part by weight of platinum. The results are shown in Table 2.

## Table 2

| Example | Number of times of catalyst used | Yield of DEMP (%) | Yield of nuclear hydrogenated product (%) | Yield of heavy matter (%) |
|---------|----------------------------------|-------------------|-------------------------------------------|----------------------------|
| 5 | 1 | 96.8 | 0.3 | 2.6 |
| 6 | 2 | 96.3 | 2.1 | 1.3 |
| 7 | 3 | 95.7 | 2.6 | 1.3 |
| 8 | 4 | 96.0 | 2.5 | 1.1 |

Note: All the conversion of m-aminophenol was 100%.

### Examples 9 to 12

The reaction of Examples 1 to 4 was repeated, except that 1.6 g of a commercially available catalyst comprising 5% by weight of platinum supported on activated carbon containing 0.15 part by weight of copper per part by weight of platinum. The results are shown in Table 3.

### Table 3

| Example | Number of times of catalyst used | Yield of DEMP (%) | Yield of nuclear hydrogenated product (%) | Yield of heavy matter (%) |
|---|---|---|---|---|
| 9 | 1 | 93.3 | 0.3 | 5.7 |
| 10 | 2 | 93.8 | 1.3 | 4.0 |
| 11 | 3 | 93.5 | 1.4 | 4.1 |
| 12 | 4 | 93.7 | 1.2 | 4.3 |

Note: All the conversion of m-aminophenol was 100%.

Comparative Examples 1 to 4

The reaction was carried out by the same operation as in Examples 1 to 4, except that 1.6 g of a catalyst comprising 5% by weight of platinum supported on activated carbon (which is a commercially available product not containing any metal element selected in the Periodic Table) was used as the catalyst. The results are as shown in Table 4.

### Table 4

| Comp. Example | Number of times of catalyst used | Yield of DEMP (%) | Yield of nuclear hydrogenated product (%) | Yield of heavy matter (%) |
|---|---|---|---|---|
| 1 | 1 | 95.5 | 1.5 | 2.4 |
| 2 | 2 | 87.7 | 8.0 | 2.0 |
| 3 | 3 | 87.1 | 7.4 | 3.0 |
| 4 | 4 | 87.5 | 7.7 | 2.6 |

Note: All the conversion of m-aminophenol was 100%.

Examples 13 to 16

3-(N,N-Di-n-butylamino)phenol was synthesized by conducting the reaction in the same manner as in Examples 1 to 4, except that 103.8 g of a methanol solution containing 50% by weight of n-butyraldehyde (n-butyraldehyde content: 0.72 mole) was used in place of the methanol solution containing 45% by weight of acetaldehyde and introduced over a period of 1 hour. The results are shown in Table 5.

Table 5

| Example | Number of times of catalyst used | Yield of DBMP (%) | Yield of nuclear hydrogenated product (%) | Yield of heavy matter (%) |
|---------|---------|---------|---------|---------|
| 13 | 1 | 95.1 | 1.3 | 1.6 |
| 14 | 2 | 94.3 | 1.9 | 1.8 |
| 15 | 3 | 94.5 | 1.8 | 1.7 |
| 16 | 4 | 94.6 | 1.7 | 1.7 |

Note:    All the conversion of m-aminophenol was 100%.

DBMP:    3-(N,N-Di-n-butylamino) phenol

Nuclear hydrogenated product:    3-(N,N-Di-n-butyl-amino)-2-hexen-1-one

Examples 17 to 20

3-(N-Cyclohexylamino)phenol was synthesized by conducting the reaction in the same manner as in Examples 1 to 4, except that 70.6 g of a methanol solution containing 50% by weight of cyclohexanone (cyclohexanone content: 0.36 mole) was used in place of the methanol solution containing 45% by weight of acetaldehyde and introduced over a period of 30 minutes. The results are shown in Table 6.

Table 6

| Example | Number of times of catalyst used | Yield of OCMP (%) | Yield of nuclear hydrogenated product (%) | Yield of heavy matter (%) |
|---------|------|------|------|------|
| 17 | 1 | 96.1 | 1.0 | 0.3 |
| 18 | 2 | 95.3 | 2.5 | 0.4 |
| 19 | 3 | 95.5 | 2.3 | 0.5 |
| 20 | 4 | 95.6 | 2.3 | 0.5 |

Note:   All the conversion of m-aminophenol was 100%.

OCMP:       3-(N-Cyclohexylamino)phenol

Nuclear hydrogenated product:       3-(N-Cyclohexyl-amino)-2-hexen-1-one

Examples 21 to 24

3-(N-Ethyl-N-isobutylamino)phenol was synthesized by following the procedure of Examples 1 to 4. In a 500 cc-volume SUS-made autoclave equipped with a stirrer were charged 32.7 g (0.30 mole) of m-aminophenol, 185.5 g of methanol, and 1.6 g of a commercially available catalyst comprising 5% by weight of platinum supported on activated carbon containing 0.1 part by weight of lead per part by weight of platinum, and 47.6 g of a methanol solution containing 50% by weight of isobutyraldehyde (isobutyraldehyde content: 0.33 mole) was introduced over a period of 30 minutes at constant conditions of 40°C and $9.8 \times 10^5$ Pa (10 kg/cm$^2$G). After completion of the introduction of isobutyraldehyde, the mixture was maintained at the same temperature for an additional 60 minutes, after which 41.1 g of a methanol solution containing 45% by weight of acetaldehyde (acetaldehyde content: 0.42 mole) and 0.20 g (0.0033 mole) of acetic acid were continuously introduced over a period of 30 minutes. After completion of the introduction of acetaldehyde, the mixture was maintained at the same temperature for an additional 70 minutes and allowed to cool. The catalyst was removed by filtration, and the resulting filtrate was analyzed by gas chromatography, liquid chromatography, and GPC. The catalyst recovered was reused in the next reaction. The results are as shown in Table 7.

Table 7

| Example | Number of times of catalyst used | Yield of EBMP (%) | Yield of nuclear hydrogenated product (%) | Yield of heavy matter (%) |
|---------|---------|---------|---------|---------|
| 21 | 1 | 95.1 | 1.2 | 1.7 |
| 22 | 2 | 94.2 | 2.1 | 1.7 |
| 23 | 3 | 94.2 | 1.9 | 1.5 |
| 24 | 4 | 94.4 | 1.9 | 1.5 |

Note:   All the conversion of m-aminophenol was 100%.

EBMP:   3-(N-Ethyl-N-isobutylamino)phenol

Nuclear hydrogenated product:   3-(N-Isobutylamino)-2-hexen-1-one

Examples 25 to 28

3-(N-Ethyl-N-isoamylamino)phenol was synthesized by following the procedure of Examples 21 to 24, except that 56.8 g of a methanol solution containing 50% by weight of isovaleraldehyde (isovaleraldehyde content: 0.33 mole) was used in place of the methanol solution containing 50% by weight of isobutyraldehyde and introduced over a period of 30 minutes. The results are shown in Table 8.

Table 8

| Example | Number of times of catalyst used | Yield of EAMP (%) | Yield of nuclear hydrogenated product (%) | Yield of heavy matter (%) |
|---------|---------|---------|---------|---------|
| 25 | 1 | 94.5 | 1.1 | 1.4 |
| 26 | 2 | 93.2 | 2.0 | 1.3 |
| 27 | 3 | 93.4 | 2.1 | 1.2 |
| 28 | 4 | 93.4 | 1.9 | 1.3 |

Note:   All the conversion of m-aminophenol was 100%.

EAMP:   3-(N-Ethyl-N-isoamylamino)phenol

Nuclear hydrogenated product:   3-(N-Isoamylamino)-2-hexen-1-one

9

Example 29

In a 500 cc-volume SUS-made autoclave equipped with a stirrer were charged 185.5 g of methanol, 1.6 g of a catalyst comprising 5% by weight of platinum supported on activated carbon, and 0.015 g of lead acetate. The mixture was stirred at room temperature for one hour. Thereafter, 32.7 g (0.30 mole) of m-aminophenol was charged, the hydrogen pressure was maintained at $9.8 \times 10^5$ Pa (10 kg/cm²), and 67.5 g of a methanol solution containing 45% by weight of acetaldehyde (acetaldehede content: 0.69 mole), and 0.20 g (0.0033 mole) of acetic acid were continuously introduced over a period of 1 hour at 40°C. After completion of the introduction of acetaldehyde, the mixture was maintained at the same temperature for an additional 90 minutes and allowed to cool. The catalyst was removed by filtration, and the resulting filtrate was analyzed by gas chromatography, liquid chromatography, and GPC. As a result, it was found to be 100% of the conversion of m-aminophenol (calculated by m-aminophenol consumed/m-aminophenol charged x 100), 94.5% of the selectivity of 3-(diethylamino)phenol, 1.7% of the selectivity of a nuclear hydrogenated product of benzene ring (nuclear hydrogenated product: 3-(diethylamino)-2-hexen-1-one), and 3.4% of the selectivity of heavy matter (values of all selectivity being relative to m-aminophenol).

Example 30

The reaction was carried out by a similar procedure to Example 29, except for using a catalyst which had been recovered after being used in Example 29 and without again treating with lead acetate. As a result of the analysis, the conversion of m-aminophenol was 100%, the selectivity of 3-(N,N-diethylamino)phenol was 94.5%, the selectivity of a nuclear hydrogenated product of benzene ring was 1.3%, and the selectivity of heavy matters was 2.9%.

Comparative Example 5

The reaction was carried out by a similar procedure to Example 29, except for using a catalyst which had been used in the reaction once without treating with lead acetate and which was not again treated with lead acetate. As a result of the analysis, the conversion of m-aminophenol was 100%, the selectivity of 3-(N,N-diethylamino)phenol was 87.8%, the selectivity of a nuclear hydrogenated product of benzene ring gas 7.2%, and the selectivity of heavy matter was 3.3%.

Comparative Example 6

The reaction was carried out by a similar procedure to Example 29, except for using the catalyst which had been recovered after being used in Comparative Example 5 and without again treating with lead acetate. As a result of the analysis, the conversion of m-aminophenol was 100%, the selectivity of 3-(N,N-diethylamino)phenol was 87.8%, the selectivity of a nuclear hydrogenated product of benzene ring was 7.0%, and the selectivity of heavy matter was 3.5%.

Example 31

The reaction was carried out by a similar procedure to Example 29, except that a catalyst prepared as follows was used: a 200 cc-volume glass-made round bottom flask was charged with 1.6 g of a catalyst comprising 5% by weight of platinum supported on activated carbon and 100 g of water to make a catalyst slurry, after which 0.015 g of lead acetate was added, and the mixture was heated at 80°C for 1 hour with stirring. After cooling the catalyst slurry, it was filtered under aspiration and then washed twice with 50 g of water, after which the solid catalyst was recovered. As a result, the conversion of m-aminophenol was 100%, the selectivity of 3-(N,N-diethylamino)phenol was 94.8%, the selectivity of a nuclear hydrogenated product of benzene ring was 2.8%, and the selectivity of heavy matter was 1.7%.

Example 32

The reaction was carried out by a similar procedure to Example 29, except for using the catalyst which had been recovered after being used in Example 31 without again treating with lead acetate. As a result of the analysis, the conversion of m-aminophenol was 100%, the selectivity of 3-(N,N-diethylamino)phenol was 95.0%, the selectivity of a nuclear hydrogenated product of benzene ring was 3.0%, and the selectivity of heavy matter was 1.5%.

### Examples 33 and 34

The reaction was carried out twice by following the procedure of Examples 29 and 30, except that the treatment was performed using copper acetate in place of the lead acetate.

### Examples 35 and 36

The reaction was carried out twice by following the procedure of Examples 29 and 30, except that the treatment was performed using zinc acetate in place of the lead acetate.

### Examples 37 and 38

The reaction was carried out twice by following the procedure of Examples 29 and 30, except that the treatment was performed using arsenic acetate in place of the lead acetate. The results of Examples 33 to 38 are shown in Table 9.

### Table 9

| Example | Yield of DEMP (%) | Yield of Nuclear Hydrogenated Product (%) | Yield of Heavy Matter (%) |
|---------|-------------------|-------------------------------------------|---------------------------|
| 33 | 92.9 | 1.7 | 4.4 |
| 34 | 92.5 | 1.3 | 3.9 |
| 35 | 92.8 | 2.8 | 2.7 |
| 36 | 93.0 | 3.0 | 2.5 |
| 37 | 91.7 | 3.5 | 3.9 |
| 38 | 92.2 | 3.0 | 4.1 |

Note: DEMP: 3-(Diethylamino)phenol

All the conversion of m-aminophenol was 100%.

### Examples 39 and 40

The reaction was carried out twice by following the procedure of Examples 29 and 30, except that 103.8 g of a methanol solution containing 50% by weight of n-butyraldehyde (n-butyraldehyde content: 0.72 mole) was used in place of the methanol solution containing 45% by weight of acetaldehyde. The results are shown in Table 10.

Table 10

| Example | Yield of DBMP (%) | Yield of Nuclear Hydrogenated Product (%) | Yield of Heavy Matter (%) |
|---|---|---|---|
| 39 | 94.5 | 1.5 | 1.7 |
| 40 | 94.3 | 1.6 | 2.0 |

Note: DBMP: 3-(N,N-Di-n-butylamino)phenol

Nuclear hydrogenated product: 3-(N,N-Di-n-butyl-amino)-2-hexen-1-one

All the conversion of m-aminophenol was 100%.


Examples 41 and 42

The reaction was carried out twice by following the procedure of Examples 29 and 30, except that 70.6 g of a methanol solution containing 50% by weight of cyclohexanone (cyclohexanone content: 0.72 mole) was used in place of the methanol solution containing 45% by weight of acetaldehyde. The results are shown in Table 11.

Table 11

| Example | Yield of OCMP (%) | Yield of Nuclear Hydrogenated Product (%) | Yield of Heavy Matter (%) |
|---|---|---|---|
| 41 | 95.5 | 2.5 | 0.4 |
| 42 | 95.7 | 2.3 | 0.5 |

Note: OCMP: 3-(N-Cyclohexylamino)phenol


Nuclear hydrogenated product: 3-(N-Cyclohexyl amino)-2-hexen-1-one

All the conversion of m-aminophenol was 100%.

Examples 43 and 44

3-(N-Ethyl-N-butylamino)phenol was synthesized by following the procedure of Examples 29 and 30. In a 500 cc-volume SUS-made autoclave equipped with a stirrer were charged 185.5 g of methanol, 1.6 g of a catalyst comprising 5% by weight of platinum supported on activated carbon, and 0.015 g of lead acetate, and the mixture was stirred at room temperature for one hour. Thereafter, 32.7 g (0.30 mole) of m-aminophenol was charged, the hydrogen pressure was maintained at 9.8 x $10^5$ Pa (10 kg/cm$^2$), and 47.6 g of a methanol solution containing isobutyraldehyde (isobutyraldehyde content: 0.33 mole) was continuously introduced over a period of 30 minutes at 40°C. After completion of the introduction of isobutyraldehyde, the mixture was maintained at the same temperature for an additional 1 hour, after which 41.1 g of a methanol solution containing 45% by weight of acetaldehyde (acetaldehyde content: 0.42 mole), and 0.20 g (0.0033 mole) of acetic acid were continuously introduced over a period of 30 minutes. After completion of the introduction of acetaldehyde, the mixture was maintained at the same temperature for an additional 70 minutes and allowed to cool. The filtrate obtained by removing the catalyst by filtration was analyzed by gas chromatography, liquid chromatography, and GPC. The results are shown in Table 12.

## Table 12

| Example | Yield of EBMP (%) | Yield of Nuclear Hydrogenated Product (%) | Yield of Heavy Matter (%) |
|---------|-------------------|-------------------------------------------|---------------------------|
| 43 | 94.2 | 2.1 | 1.7 |
| 44 | 94.5 | 1.9 | 1.5 |

Note:　EBMP:　3-(N-Ethyl-N-isobutylamino)phenol

　　　Nuclear hydrogenated product:　　3-(N-Isobutylamino)-2-hexen-1-one

All the conversion of m-aminophenol was 100%.

### Examples 45 and 46

The reaction was carried out twice by following the procedure of Examples 43 and 44, except that 56.8 g of a methanol solution containing 50% by weight of isovaleraldehyde (isovaleraldehyde content: 0.33 mole) was used in place of the methanol solution containing 50% by weight of n-butyraldehyde. The results are shown in Table 13.

Table 13

| Example | Yield of EAMP (%) | Yield of Nuclear Hydrogenated Product (%) | Yield of Heavy Matter (%) |
|---------|-------------------|-------------------------------------------|---------------------------|
| 45 | 93.5 | 2.2 | 1.4 |
| 46 | 93.7 | 2.0 | 1.4 |

Note: EAMP: 3-(N-Ethyl-N-isoamylamino)phenol

Nuclear hydrogenated product: 3-(N-Isoamylamino)-

2-hexen-1-one

All the conversion of m-aminophenol was 100%.

**Claims**

1. A process for preparing an N-alkylaminophenol or N,N-dialkylamimophenol, which comprises subjecting an aminophenol to reductive alkylation with an aldehyde or a ketone in the presence of an organic solvent, a catalyst for reduction, and hydrogen, wherein said catalyst for reduction comprises (a) platinum and at least one metal element selected from metal elements belonging to the IB group, IIB group, IVB group, VB group and VIB group of the Periodic Table, supported on activated carbon, or comprises (b) palladium and at least one metal element selected from metal elements belonging to the IB group, IIB group, IVB group, VB group and VIB group of the Periodic Table, supported on activated carbon.

2. A process as claimed in Claim 1, wherein the catalyst contains the metal element selected from the Periodic Table in an amount in the range of from 0.001 to 0.5 parts by weight per part of platinum or palladium.

3. A process as claimed in Claim 1 or 2, wherein the metal element selected from the Periodic Table is lead, tellurium, copper or arsenic.

**Patentansprüche**

1. Verfahren zur Herstellung eines N-Alkylaminophenols oder N,N-Dialkylaminophenols, das die reduktive Alkylierung eines Aminophenols mit einem Aldehyd oder einem Keton in Gegenwart eines organischen Lösungsmittels, eines Reduktionskatalysators und Wasserstoff umfaßt, wobei der Reduktionskatalysator (a) Platin und wenigstens ein metallisches Element ausgewählt aus den metallischen Elementen der IB-Gruppe, IIB-Gruppe, IVB-Gruppe, VB-Gruppe und VIB-Gruppe des Periodensystems, die auf Aktivkohle aufgebracht sind, umfaßt, oder (b) Palladium und wenigstens ein metallisches Element ausgewählt aus den metallischen Elementen der IB-Gruppe, IIB-Gruppe, IVB-Gruppe, VB-Gruppe und VIB-Gruppe des periodischen Systems, die auf Aktivkohle aufgebracht sind, umfaßt.

2. Verfahren nach Anspruch 1, wobei der Katalysator das metallische Element ausgewählt aus dem Periodensystem in einer Menge im Bereich von 0,001 bis 0,5 Gew.-Teilen pro Gew.-Teil Platin oder Palladium enthält.

3. Verfahren nach Anspruch 1 oder 2, wobei das metallische Element ausgewählt aus dem periodischen System Blei, Tellur, Kupfer oder Arsen ist.

**Revendications**

1. Procédé pour la préparation d'un N-alkylaminophénol ou d'un N,N-dialkylaminophénol, qui consiste à soumettre un aminophénol à une alkylation réductrice avec un aldéhyde ou une cétone en présence d'un solvant organique, d'un catalyseur de réduction et d'hydrogène, dans lequel ledit catalyseur de réduction comprend (a) du platine et au moins un élément métallique choisi parmi les éléments métalliques appartenant au groupe IB, au groupe IIB, au groupe IVB, au groupe VB et au groupe VIB du tableau périodique des éléments supportés sur du charbon actif, ou comprend (b) du palladium et au moins un élément métallique choisi parmi les éléments métalliques appartenant au groupe IB, au groupe IIB, au groupe IVB, au groupe VB et au groupe VIB du tableau périodique des éléments, supportés sur du charbon actif.

2. Procédé selon la revendication 1, dans lequel le catalyseur contient l'élément métallique choisi dans le tableau périodique des éléments en une quantité dans la gamme de 0,001 à 0,5 partie en poids par partie de platine ou de palladium.

3. Procédé selon la revendication 1 ou 2, dans lequel l'élément métallique choisi dans le tableau périodique est le plomb, le tellure, le cuivre ou l'arsenic.